Europäisches Patentamt

European Patent Office

Office européen des brevets

(19)

(11) Numéro de publication: **0 441 695 B1**

# (12) FASCICULE DE BREVET EUROPEEN

(45) Date de publication de fascicule du brevet: **24.05.95**

(51) Int. Cl.6: **C12P 21/02**, C07K 14/755,
//C12N5/06

(21) Numéro de dépôt: **91400267.0**

(22) Date de dépôt: **05.02.91**

(54) **Procédé pour la préparation du facteur VIII humain et d'analogues du facteur VIII.**

(30) Priorité: **05.02.90 FR 9001302**

(43) Date de publication de la demande:
**14.08.91 Bulletin 91/33**

(45) Mention de la délivrance du brevet:
**24.05.95 Bulletin 95/21**

(84) Etats contractants désignés:
**AT BE CH DE DK ES FR GB GR IT LI LU NL SE**

(56) Documents cités:
**EP-A- 53 046
EP-A- 98 256
EP-A- 254 076
EP-A- 306 968
WO-A-86/05190**

(73) Titulaire: **TM INNOVATION
58 avenue Leclerc
F-69007 Lyon (FR)**

(72) Inventeur: **Mignot, Gérard
9 Résidence Guinconces
91190 Gif S/Yvette (FR)**
Inventeur: **Bihoreau, Nicolas
11 Résidence les Rieux
91120 Palaiseau (FR)**
Inventeur: **Adamowicz, Philippe
16 Allées des Haras
92420 Vaucresson (FR)**

(74) Mandataire: **Warcoin, Jacques et al
Cabinet Régimbeau,
26, avenue Kléber
F-75116 Paris (FR)**

EP 0 441 695 B1

Rank Xerox (UK) Business Services
(3.10/3.09/3.3.3)

**Description**

La présente invention concerne un procédé pour la préparation du facteur VIII humain et d'analogues du facteur VIII.

Le facteur VIII est un facteur de la coagulation sanguine qui est utilisé pour le traitement des patients atteints d'hémophilie A.

Le facteur VIII utilisé dans ce traitement est actuellement essentiellement constitué de concentrés de facteur VIII obtenus par fractionnement à partir de plasma humain. Depuis, on a cherché à remplacer cette source de facteur VIII par une source plus facilement accessible et moins sujette aux contaminations virales ou autres, c'est pourquoi un effort important a été entrepris afin d'obtenir le facteur VIII par génie génétique. Les résultats obtenus actuellement montrent que le facteur VIII de recombinaison présente toutes les caractéristiques du facteur VIII naturel et qu'il peut être obtenu dans des conditions industrielles très satisfaisantes.

La préparation de facteur VIII par des procédés mettant en oeuvre des techniques de génie génétique a déjà été très largement décrite notamment dans les brevets suivants :

- EP-A-162 067,
- EP-A-182 448,
- EP-A-150 735,
- EP-A-157 556, et
- EP-A-160 457.

Notamment, le brevet EP-A-162 067 mentionne l'expression du facteur VIII dans différents types de cellules eucaryotes, en particulier des cellules CHO.

Le brevet EP-A-160 457 décrit également l'expression du facteur VIII dans des cellules eucaryotes, en particulier des cellules BHK.

Les brevets FR-A-86 08258 et FR-A-87 04699 décrivent la préparation de facteur VIII en utilisant des cellules eucaryotes et le virus de la vaccine comme système d'expression. Afin de conduire à une production optimum de facteur VIII, le milieu de culture contient le facteur Von Willebrand.

A titre de cellules eucaryotes, le brevet EP-A-253 455 suggère également d'utiliser des levures pour préparer du facteur VIII.

De nombreux analogues du facteur VIII ont également été proposés. Parmi les brevets traitant de ces dérivés, on peut tout particulièrement citer :

- WO-A-86 06101,
- EP-A-232 112,
- WO-A-87 07144,
- WO-A-88 00381,
- EP-A-265 778,
- EP-A-294 910, et
- EP-A-303 540.

Ces analogues du facteur VIII présentent divers avantages sur le "facteur VIII naturel" et peuvent être comme lui obtenus par des techniques de génie génétique.

Les procédés actuellement retenus utilisent de préférence des cellules eucaryotes exprimant le facteur VIII, soit qu'il s'agisse de cellules ayant intégré une ou plusieurs séquences codantes pour le facteur VIII au niveau chromosomique, soit qu'il s'agisse de cellules incorporant des vecteurs viraux exprimant le facteur VIII.

Ce type de technologie est largement décrit dans les brevets précités et ne sera pas redécrit ici en détail. Il sera simplement fait mention des cellules exprimant le facteur VIII ou un analogue du facteur VIII.

Par analogue du facteur VIII, on entendra désigner une molécule ayant l'activité du facteur VIII et obtenue à partir de facteur VIII par délétion de certains aminoacides ou encore une molécule de facteur VIII ayant subi certaines mutations potentielles mais qui conserve néanmoins l'activité principale du facteur VIII.

Le document EP-A-53 046 décrit un procédé de stabilisation de l'activité du facteur VIII dans le plasma sanguin frais qui vient d'être collecté par addition d'un agent anticoaguiant chélatant du calcium et d'héparine dissoute en quantité de l'ordre de 2 à 20 unités par ml pour l'héparine et de façon que la concentration d'ions calcium libre soit d'environ de 5 mg de calcium pour 100 ml de plasma.

Par ailleur, le document EP-A-98 256 décrit une méthode de préparation d'un concentré de facteur VIII:C à partir de plasma sanguin comprenant le fractionnement du plasma sanguin au moyen d'étapes d'adsorption mettant en jeu deux copolymères polyélectrolytes réticulés différents insolubles dans l'eau, chacun étant ajouté en présence d'héparine exogène.

Comme cela est décrit dans les brevets WO-87 04187, FR-A-8608258 et FR-A-87 04699, il peut être intéressant, afin d'optimiser la préparation de facteur VIII ou d'un analogue du facteur VIII, de prévoir l'addition dans le milieu de culture de cellules du facteur Von Willebrand ou bien de phospholipide.

Il a en effet été montré que la présence de ce facteur Von Willebrand dans le milieu de culture permettait de stabiliser le facteur VIII et d'améliorer considérablement le rendement.

Pour des raisons similaires à celles qui viennent d'être mentionnées, le facteur Von Willebrand utilisé est bien entendu un facteur Von Willebrand recombinant afin d'éviter tout risque de contamination. Ceci nécessite la mise en oeuvre d'une étape supplémentaire de fabrication et une augmentation de coût du procédé de préparation du facteur VIII, notamment pratiquement un doublement du prix.

Il serait donc intéressant de disposer d'un procédé de préparation du facteur VIII ne nécessitant pas l'utilisation de facteur Von Willebrand mais conduisant malgré tout à des rendements importants en facteur VIII.

L'objet de la présente invention est précisément de proposer une solution à ce problème.

Plus particulièrement, la présente invention concerne un procédé de préparation de facteur VIII ou d'un analogue du facteur VIII par culture de cellules eucaryotes ayant incorporé un vecteur d'expression contenant un gène codant pour ledit facteur VIII ou un analogue de facteur VIII et séparation du facteur VIII ou de son analogue, caractérisé en ce que le milieu de culture contient au moins un dérivé d'un polymère polyanionique polysulfaté et en ce qu'il est exempt de sérum.

Parmi les analogues du facteur VIII on citera tout particulièrement le facteur VIII delta 2 qui correspond a une délétion des acides aminés 771 à 1666 du facteur VIII et dont la préparation est plus particulièrement décrite dans le brevet EP 303 540.

Les polymères selon la présente invention sont de préférence des dérivés de polymères polyosidiques notamment de polysaccharides. Ces polymères sont de préférence sulfatés.

Ce type de polymères est connu, il s'agit notamment de produits obtenus par fermentation bactérienne par exemple le dextran sulfaté ultérieurement par voie chimique, ou bien il peut s'agir de produits d'extraction naturellement sulfatés tels que les mucopolysaccharides de type héparine, héparine de bas poids moléculaire ou héparinoïdes, héparane sulfate, dermatane sulfate.

Le sulfate d'hydroxyéthyl d'amidon peut également être utilisé à titre de polymère.

Ces composés sont particulièrement intéressants compte tenu de leur faible coût et de leur grande disponibilité.

Le polymère utilisé selon la présente invention a un poids moléculaire compris entre 1000 et 1 000 000.

Selon un mode de réalisation particulier de l'invention, de polymère utilisé est un dextran sulfaté. Le degré de sulfatation de ce dextran sulfaté est de l'ordre de 0,5 % à environ 18 % en poids en soufre et de préférence de 10 à environ 18 %.

Des dextran sulfatés de poids moléculaire très divers peuvent être utilisés selon l'invention. Leur poids peut ainsi varier de 5 000 à 700 000 Daltons.

La présence d'un polymère polyanionique selon l'invention, dans un milieu de culture approprié pour la production de facteur VIII ou analogue du facteur VIII et exempt de facteur Von Willebrand conduit donc à des rendements en facteur VIII ou analogue du facteur VIII nettement supérieurs à ceux obtenus dans un milieu ne contenant ni de facteur Von Willebrand ni de polymère selon l'invention.

Ainsi, il ressort des expériences réalisées selon le procédé de la présente invention et décrites dans des exemples ci-après, qu'il est possible, à partir d'un milieu de culture à base de dextran sulfaté de poids moléculaire 500 000, d'obtenir des quantités importantes de facteur VIII delta 2 mesuré par deux méthodes différentes, l'une basée sur une méthode immunoenzymatique, mettant en jeu des anticorps monoclonaux anti-facteur VIII, et l'autre par une méthode physico-chimique telle que l'analyse par PAGE-SDS en conditions réductrices ou non mettant en évidence une bande majeure à 165 KDa qui représente la forme majeure du facteur VIII delta 2 exprimée en l'absence de sérum de veau.

Des poids moléculaires (5000, 8000, 15 000, 50 000, 500 000 Da) ont donné des résultats sensiblement équivalents sur l'expression du facteur VIII.

Le milieu de culture supplémenté avec un polymère polyanionique peut bien entendu contenir d'autres agents susceptibles de favoriser la production du facteur VIII. Il peut s'agir d'agents antiprotéasiques et/ou de chlorure de calcium connu pour ses propriétés stabilisantes.

Dans ces conditions, il a été montré que, pour un milieu de culture supplémenté avec 1000 mg/l de dextran sulfaté et 2 mmoles de chlorure de calcium (1 mM dans le milieu de base + 1 mM rajoutée), il est possible d'accumuler en 24 heures des quantités de facteur VIII delta 2 mesurées par une méthode immunoenzymatique 6 à 7 fois plus importantes que la quantité accumulée en l'absence de facteur Von Willebrand exogène.

La molécule de facteur VIII complète ou delétée de la région B a la particularité de présenter de fortes densités de charges très localisées créant des pôles chargés positivement et négativement capables de se lier par des forces de Coulomb (interactions de type ionique) à des polyanions et à des polycations respectivement. L'effet bénéfique du dextran sulfaté sur la production de facteur VIII peut s'expliquer par l'existence de telles interactions entre les groupements sulfates du dextran et les aminoacides basiques du facteur VIII. Cette stabilisation de la molécule du facteur VIII complète ou délétée est en outre à associer a une augmentation du taux d'expression du facteur VIII ou dérivé du facteur VIII.

Il y a vraisemblablement formation d'un complexe entre la molécule de facteur VIII complète ou délétée et au moins une des molécules du dérivé polyanionique mis en oeuvre dans le milieu de culture.

La présente invention se rapporte également à cette forme complexée du facteur VIII ou dérivé du facteur VIII.

Cette forme stabilisée du facteur VIII ou d'un de ses dérivés peut présenter un grand intérêt en particulier pour de stockage dudit facteur VIII ou dérivé du facteur VIII au cours du procédé de production ou à la fin de celui-ci. De plus, lorsque le facteur VIII ou l'un de ses dérivés est complexé à du dextran sulfaté qui est un agent de lyophilisation, on peut espérer que le complexe ainsi formé présentera également une bonne aptitude à la lyophilisation.

La présence de dextran sulfaté peut donc avoir à la fois un effet stabilisant vis-à-vis du facteur VIII et également un effet de stimulation sur l'expression du facteur VIII par interaction au niveau des membranes cellulaires.

Le milieu de culture utilisé selon l'invention est un milieu de culture sans sérum, de préférence à base notamment d'insuline humaine recombinante et pouvant contenir d'autres protéines exogènes.

Ces protéines sont de préférence utilisées à une concentration de l'ordre de 4 mg/l.

Les cellules utilisées pour la production du facteur VIII ou analogue du facteur VIII sont de préférence des cellules de mammifères et plus particulièrement des cellules CHO recombinées exprimant le facteur VIII ou analogue du facteur VIII de façon permanente.

Ces cellules sont transfectées par un vecteur d'intégration comportant l'ADNc du facteur VIII ou dérivé du facteur VIII entouré des éléments nécessaires à son expression dans les cellules eucaryotes et un segment d'ADN favorisant l'intégration du vecteur dans l'ADN cellulaire. Il est ainsi possible d'utiliser à titre de vecteur d'expression du facteur VIII le pTG 1020 et du facteur VIII delta II le pTG 1509 décrits dans le brevet EP 0 303 540 de même que des vecteurs d'expression de constructions équivalentes. Les souches des plasmides pTG 1509 et pTG 1020 ont été déposées sous le N° 1-679 et 1-681 à la Collection Nationale de Culture des Microorganismes.

La transfection des cellules CHO par ces vecteurs d'intégration est réalisée selon la technique décrite dans le brevet EP 0 303 540 et ne sera pas rappelée ici.

Le procédé selon l'invention présente donc de nombreux avantages.

Il permet d'obtenir le facteur VIII ou analogue du facteur VIII par génie génétique avec un rendement optimisé.

Il s'affranchit de la présence de facteur Von Willebrand dans le milieu de culture et par là même conduit à un gain important de temps et de coût pour la production industrielle de facteur VIII ou analogue du facteur VIII.

Enfin, certains types de polymère utilisé selon l'invention tel que le sulfate dextran n'entraine aucun risque de contamination virale ou autres, est peu onéreux, et facile à se procurer pour l'homme du métier.

D'autres avantages et caractéristiques de la présente invention apparaîtront à la lecture des exemples présentés ci-dessous à titre non limitatif de celle-ci.

**PARTIE EXPERIMENTALE**

Les études des effets des polymères polyosidiques sulfatés d'une part sur l'expression du facteur VIII delta 2, ont été réalisées à partir du clone CHO TG2307-11-3 (exemples 1 à 4) et d'autre part sur l'expression du facteur VIII complet à partir du clone CHO TG1566-3013 (exemples 5 et 6).

EXEMPLE 1

Etude des effets du dextran T 2000 sur l'expression du facteur VIII delta 2

Dans un premier temps à titre de référence, les effets du dextran T2000 (PHARMACIA PM: 2 000 000 Da) ont été évalués. Le tableau n° 1 ci-après résume les résultats obtenus comparativement à un milieu contenant 2 U/ml de facteur Von Willebrand recombinant.

Le dextran T 2000 (non sulfaté) est sans effets sur l'expression du facteur VIII delta 2 en l'absence de sérum et de facteur Von Willebrand recombinant.

Par conséquent, la matrice de dextran constituée d'unités glucopyranosyles non substituées n'interfère pas avec la molécule de facteur VIII delta 2.

Tableau 1

| ETUDE DES EFFETS DE L'ADDITION DU DEXTRAN T 2000 MILIEUX DE PRODUCTION DU FACTEUR VIII DELTA 2 CLONE CHO TG 2307-11-3 | | |
|---|---|---|
| MILIEUX DE PRODUCTION | APTT mU/ML J3-J4 | MOYENNE |
| I/H INS HUM 4 mg/L + 2 U vWFrec CNTS/ML | 1000 | |
| | 1000 | 1000 |
| I/H INS HUM 4 mg/L + 0 mg/L DEXTRAN T2000 | 250 | |
| | 280 | 265 |
| I/H INS HUM 4 mg/L + 1000 mg/L DEXTRAN T2000 | 250 | |
| | 320 | 300 |
| I/H INS HUM 4 mg/L + 2000 mg/L DEXTRAN T2000 | 265 | |
| | 300 | 280 |
| PASSAGE P15 POST DECONGELATION ET P12 EN MEM HT CROISSANCE (37) ET ESSAIS DE PRODUCTION (35C) EN CHANGEMENT DE MILIEU TOUTES LES 24 HEURES INS HUMAINE ELI LILLY 319KK9B DEXTRAN T 2000 (PHARMACIA): PM 2 000 000 Da | | |

**EXEMPLE 2 :**

**Etude des effets de polymères sulfatés sur l'expression de facteur VIII delta 2**

1) Le dextran sulfaté

Un effet dose du dextran sulfaté (PHARMACIA PM:500 000) a été effectué. Le tableau n° 2 présenté ci-après rend compte également des données obtenues à partir du même milieu contenant deux unités de facteur Von Willebrand recombinant.

Dans ces conditions, il est possible, dans un cycle de production, qui dure du 3ème au 6ème jour avec renouvellement du milieu chaque jour, d'obtenir sur une des périodes d'incubation, (5ème au 6ème jour) des quantités de facteur VIII delta 2, mesurées en ELISA, équivalentes au maximum aux 2/3 des activités accumulées en présence de deux unités/ml de facteur Von Willebrand recombinant.

Ces activités représentent en outre environ 2 à 3 fois le niveau des activités obtenues dans un milieu ne contenant ni facteur Von Willebrand recombinant, ni de dextran sulfaté et cela en absence de phospholipides.

2) L'héparine bovine (KABI VITRUM)

Les résultats d'une étude d'un effet-dose héparine bovine sont résumés dans le tableau n° 3.

Sur les premiers cycles de production, l'héparine montre un effet très limité voir nul sur l'expression du facteur VIII delta 2. Sur le dernier jour d'incubation J5-J6 une relation effet-dose très faible peut être mise en évidence.

Tableau 2

| ETUDE DES EFFETS DE L'ADDITION DE SULFATE DE DEXTRAN DANS LES MILIEUX DE PRODUCTION DU FACTEUR VIII DELTA 2 CLONE CHO TG 2307-11-3 | | | | | | |
|---|---|---|---|---|---|---|
| MILIEUX DE PRODUCTION | APTT mU/ML J3-J4 | ELISA mU/ml J3-J4 | APTT mU/ML J4-J5 | ELISA mU/ML J4-J5 | APTT mU/ML J5-J6 | ELISA mU/ML J5-J6 |
| MEM HT 10 SVF ND | 570 | | - | 11700 | 720 | 15900 |
| | 608 | | 651 | 9000 | 760 | 16400 |
| I/H INS HUM 4 mg/L + 2 U vWFrec CNTS/ML | 249 | | 467 | 7300 | 500 | 10000 |
| | 280 | | 409 | 7500 | 510 | 8400 |
| I/H INS HUM 4 mg/L + 250 mg/L DS | ND | 2800 | ND | 2200 | ND | 4200 |
| | | 6000 | | 1600 | | 4500 |
| I/H INS HUM 4 mg/L + 500 mg/L DS | ND | 4500 | ND | 4600 | ND | 4600 |
| | | 3100 | | 4300 | | 4300 |
| I/H INS HUM 4 mg/L + 1000 mg/L DS | ND | 3400 | ND | 4800 | ND | 5000 |
| | | 3000 | | 4700 | | 6400 |
| I/H INS HUM 4mg/L + 2000 mg/L DS | ND | 3800 | ND | 5000 | ND | 6300 |
| | | 3400 | | 5000 | | 6500 |
| PASSAGE P24 POST DECONGELATION ET P21 EN MEM HT SVF ND 701121 | | | | | | |
| CROISSANCE (37) ET ESSAIS DE PRODUCTION (35C) EN PLAQUES 6 PUITS NUNC | | | | | | |
| CHANGEMENT DE MILIEU TOUTES LES 24 HEURES | | | | | | |
| INS HUMAINE ELI LILLY 319KK9B | | | | | | |
| PLAQUES 6 PUITS NUNC | | | | | | |
| DS: DEXTRAN SULFATE PM: 500 000 PHARTMACIA 17% S | | | | | | |
| ND = NON DOSABLE DU FAIT DU POUVOIR ANTICOAGULANT DU DEXTRAN SULFATE | | | | | | |

Tableau 3

| ETUDE DES EFFETS DE L'ADDITION D'HEPARINE BOVINE DANS LES MILIEUX DE PRODUCTION DU FACTEUR VIII DELTA 2 CLONE CHO TG 2307-11-3 | | | | | | |
|---|---|---|---|---|---|---|
| MILIEUX DE PRODUCTION | APTT mU/ML J3-J4 | ELISA mU/ml J3-J4 | APTT mU/ML J4-J5 | ELISA mU/ML J4-J5 | APTT mU/ML J5-J6 | ELISA mU/ML J5-J6 |
| MEM HT 10 SVF ND | 1500<br><br>1570 | 18530<br><br>14920 | 1920<br><br>2230 | 35000<br><br>36000 | 5000<br><br>2830 | 36000<br><br>27300 |
| I/H INS HUM 4 mg/L + 2 U vWFrec CNTS/ML | 900<br><br>1050 | 11410<br><br>11400 | 1220<br><br>1070 | 24400<br><br>29300 | 1870<br><br>2100 | 28900<br><br>32500 |
| I/H INS HUM 4 mg/L | 213<br>148 | 1850<br>1460 | 130<br>110 | 2630<br>3050 | 142<br>125 | 2000<br>1100 |
| I/H INS HUM 4 mg/L HEPARINE 10 U/ML | ND | 2060<br><br>1300<br>2950 | ND | 1110<br><br>1560<br>1850 | ND | 800<br><br>1100<br>2400 |
| I/H INS HUM 4 mg/L HEPARINE 20 U/ML | ND | 2430<br><br>2300<br>2480 | ND | 1650<br><br>2060<br>1940 | ND | 2400<br><br>3000<br>3400 |
| I/H INS HUM 4mg/L HEPARINE 40 U/ML | ND | 2500<br><br>3620<br>3120 | ND | 2170<br><br>3220<br>2350 | ND | 3500<br><br>5300<br>4800 |
| I/H INS HUM 4mg/L HEPARINE 80U/ML | ND | 3120<br><br>3680<br>3920 | ND | 2850<br><br>3400<br>3400 | ND | 4000<br><br>5300<br>5000 |
| PASSAGE P24 POST DECONGELATION ET P21 EN MEM HT SVF ND 701121<br>CROISSANCE (37) ET ESSAIS DE PRODUCTION (35C) EN PLAQUES 6 PUITS NUNC<br>CHANGEMENT DE MILIEU TOUTES LES 24 HEURES<br>INS HUMAINE ELI LILLY 319KK9B<br>HEPARINE BOVINE KABI VITRUM<br>ND = NON DOSABLE DU FAIT DU POUVOIR ANTICOAGULANT DE L'HEPARINE | | | | | | |

**EXEMPLE 3 :**

Il a été également évalué les effets de combinaison de dextran sulfaté et de chlorure de calcium sur le taux d'expression du facteur VIII delta 2 comparativement à des milieux témoins contenant soit 10 % de sérum de veau foetal, soit 2U/ml de facteur von Willebrand, soit uniquement de l'insuline humaine qui rentre dans la composition de tous les milieux sans sérum utilisés dans l'expérience.

Les résultats obtenus sont présentés dans le tableau n° 4 ci-après.

Il ressort que pour les points qui sont proches de l'optimum c'est-à-dire 1000 mg/l de dextran sulfaté et 2 mM de chlorure de calcium (1 mM dans le milieu de base + 1 mM rajoutée), il est possible d'accumuler en 24 heures entre 20 et 21 unités d'antigène de facteur VIII delta 2 soit environ les 2/3 de la quantité accumulée en présence de 2 unités de facteur Von Willebrand recombinant (2U/ml) et 6 à 7 fois la quantité accumulée en l'absence de sérum de veau foetal ou de facteur Von Willebrand.

On remarque également qu'en l'absence de sérum de veau foetal ou de facteur Von Willebrand recombinant, les activités accumulées en 24 heures ne dépassent jamais 3 unités/ml et sont en moyenne de 2,27 unités/ml, soit très significativement inférieures aux activités accumulées en présence de quantités optimales de dextran sulfaté.

Tableau 4

ETUDE DES EFFETS DE L'ADDITION DE SULFATE DE DEXTRAN DANS LES
MILIEUX DE PRODUCTION DU FACTEUR VIII DELTA 2
CLONE CHO TG 2307-11-3
DOEHLERT DEXTRAN SULFATE (0-2000 mg/L)/ CaCl2 (2-11mM)

| MILIEUX DE PRODUCTION | APTT mU/ML J3-J4 | ELISA mU/ml J3-J4 | APTT mU/ML J4-J5 | ELISA mU/ML J4-J5 | APTT mU/ML J5-J6 | ELISA mU/ML J5-J6 | APTT mU/ML J6-J7 | ELISA mU/ML J6-J7 |
|---|---|---|---|---|---|---|---|---|
| MEM HT 10% SVF ND | 1500 | 18500 | 1920 | 34000 | 2830 | 28000 | 1310 | 30700 |
| | 1600 | 14900 | 2230 | 35000 | 5000 | 36000 | 1850 | 35000 |
| L/H INS HUM 4 mg/L + 2 U vWFrec CNTS/ML | 1050 | 11400 | 1220 | 24400 | 1870 | 28900 | 1750 | 33700 |
| | 900 | 11400 | 1070 | 29300 | 2100 | 32500 | 1370 | 26700 |
| L/H INS HUM 4 mg/L | 210 | 1850 | 130 | 2650 | 125 | 1130 | 142 | 3000 |
| | 150 | 1460 | 110 | 3050 | 142 | 1900 | 124 | 3170 |
| * L/H INS HUM 4 mg/L + 1000 mg/L DS Ca 5mM | ND | 856 | ND | 8000 | ND | 5000 | ND | 5700 |
| L/H INS HUM 4 mg/L + 1000 mg/L DS Ca 10mM | ND | 980 | ND | 6150 | ND | 4700 | ND | 3920 |
| | | | | | | 6000 | | 4980 |
| L/H INS HUM 4 mg/L + 1000 mg/L DS Ca 1 mM | ND | 1350 | ND | 8400 | ND | - | ND | 21610 |
| | | | | | | - | | 20550 |
| L/H INS HUM 4mg/L + 1866 mg/L DS Ca 7.5 mM | ND | 1380 | ND | 8550 | ND | 10700 | ND | 12000 |
| | | | | | | 8000 | | 10520 |
| L/H INS HUM 4 mg/L + 134 mg/L DS Ca 2.5 mM | ND | 655 | ND | 6600 | ND | 9900 | ND | 15585 |
| | | | | | | 10150 | | 15410 |
| L/H INS HUM 4 mg/1 + 134 mg/L DS Ca 7.5 mM | ND | 250 | ND | 6700 | ND | 7800 | ND | 11260 |
| | | | | | | 10150 | | 13900 |
| L/H INS HUM 4 mg/L + 1866 mg/L DS Ca 2.5 mM | ND | 1420 | ND | 6300 | ND | 9700 | ND | 15510 |
| | | | | | | 10950 | | 14120 |

PASSAGE P24 POST DECONGELATION ET P21 EN MEM HT SVF ND 701121
CROISSANCE (37) ET ESSAIS DE PRODUCTION (35C) EN PLAQUES 6 PUITS NUNC
CHANGEMENT DE MILIEU TOUTES LES 24 HEURES
INS HUMAINE ELI LILLY 319X09B
PLAQUES 6 PUITS NUNC
DS= DEXTRAN SULFATE PM:500 000 PHARMACIA 17% S
*= MOYENNE DE 6 PUITS (BO) AUTRES VALEURS MOYENNE DE 2 PUITS
ELISA J3-J4 FAIT 24 H APRES PRELEVEMENT
ND: NON DOSABLE DU FAIT DU POUVOIR ANTICOAGULANT DU DEXTRAN SULFATE

Tableau 5

ETUDE DES EFFETS DE L'ADDITION DE POLYMERES SULFATES DANS LES
MILIEUX DE PRODUCTION DU FACTEUR VIII DELTA 2
CLONE CHO TG 2307-11-3
EFFETS DE SULFATES D'HYDROXYETHYLAMIDON DE PM 200 000 Da A 3.76% ET 13.1% DE S
ET DE CARBOXYMETHYLCELLULOSE LOW VISCOSITY SIGMA

| MILIEUX DE PRODUCTION | APTT mU/ML J3-J4 | ELISA mU/ml J3-J4 | APTT mU/ML J4-J5 | ELISA mU/ML J4-J5 | APTT mU/ML J5-J6 | ELISA mU/ML J5-J6 | APTT mU/ML J6-J7 | ELISA mU/ML J6-J7 |
|---|---|---|---|---|---|---|---|---|
| MEM HT 10% SVF ND | 3100 | 40200 | 1370 | 20000 | 740 | 18500 | 810 | 13500 |
| I/H INS HUM 4 mg/L + 2 U vWFrec CNTS/ML | 1300 | 18200 | 1530 | 30800 | 940 | 22400 | 1600 | 21000 |
| I/H INS HUM 4 mg/L | 170 | 3200 | 124 | 1100 | 25 | 760 | 260 | 1900 |
| I/H INS HUM 4 mg/L + 100 mg/L CMC | ND | 5500 | ND | 1400 | ND | 970 | ND | 1820 |
| I/H INS HUM 4 mg/L + 500 mg/L CMC | ND | 4600 | ND | 1900 | ND | 850 | ND | 2100 |
| I/H INS HUM 4 mg/L + 1000 mg/L CMC | ND | 4400 | ND | 2200 | ND | 1200 | ND | 2350 |
| I/H INS HUM 4mg/L + 100 mg/L SHEA 13.1% | ND | 8700 | ND | 10300 | ND | 5800 | ND | 5700 |
| I/H INS HUM 4 mg/L + 500 mg/L SHEA 13.1% | ND | 12800 | ND | 21900 | ND | 9900 | ND | 10500 |
| I/H INS HUM 4mg/L + 1000 mg/L SHEA 13.1% | ND | 11900 | ND | 18000 | ND | 11500 | ND | 12800 |
| I/H INS HUM 4 mg/L + 100 mg/L SHEA 3.76% | ND | 7500 | ND | 4900 | ND | 2100 | ND | 2240 |
| I/H INS HUM 4 mg/l + 500 mg/L SHEA 3.76% | ND | 8300 | ND | 11000 | ND | 5000 | ND | 4740 |
| I/H INS HUM 4 mg/L + 1000 mg/L SHEA 3.76% | ND | 8200 | ND | 14200 | ND | 6700 | ND | 5530 |

PASSAGE P19 POST DECONGELATION ET P18 EN MEM HT SVF ND 701121
CROISSANCE (37) ET ESSAIS DE PRODUCTION (35C) EN PLAQUES 6 PUITS NUNC
CHANGEMENT DE MILIEU TOUTES LES 24 HEURES
INS HUMAINE ELI LILLY 319KK9B
PLAQUES 6 PUITS NUNC
SULFATE D'HYDROXYETHYLAMIDON PM:200 000 PFEIFER ET LANGEN 3.76 ET 13.1% S
vWFrec CNTS CONCENTRE A 63.7 U/ML DU 11/12/89
ND: NON DOSABLE DU FAIT DU POUVOIR ANTICOAGULANT DES POLYMERES SULFATES
ELISA DE J4-J5: INCUBATION Ag 14 HEURES A+20°C

## EXEMPLE 4

D'autres polymères et en particulier la carboxyméthylcellulose, le sulfate d'hydroxyéthylamidon (PM : 200 000) à 3,76 et 13,1 % de soufre ont été évalués pour leur capacité à influencer le taux d'expression du facteur VIII delta 2 dans un milieu sans sérum et sans facteur Von Willebrand et ce, par rapport à des témoins contenant 10 % de sérum bovin, 2 unités/ml de facteur Von Willebrand recombinant ou ni sérum ni Von Willebrand.

La carboxyméthylcellulose aux concentrations testées n'a pas d'effets marqués sur l'expression du facteur VIII delta 2, alors que le sulfate d'hydroxyéthylamidon à 13 % de soufre à des concentrations de 0,5 et 1,0 g/l permet d'accumuler en 24 heures jusqu'à environ 20 unités C : Ag/ml de facteur VIII delta 2 soit environ les 2/3 de ce qui est obtenu dans le même temps en présence de 2 unités/ml de facteur Von Willebrand recombinant. Le même polymère ne contenant que 3,76 % de soufre a un effet sensiblement moins marqué sur l'expression du facteur VIII delta 2. Cette expérience confirme l'intérêt des polysaccharides sulfatés et révèle l'importance du degré de sulfatation dans l'intéraction des polymères avec la molécule de facteur VIII delta 2.

**EXEMPLE 5**

**Etude des effets du dextran sulfaté sur l'expression du facteur VIII**

L'étude est réalisée à partir du clone CHO TG 1566-3013 qui exprime la molécule du facteur VIII complet.

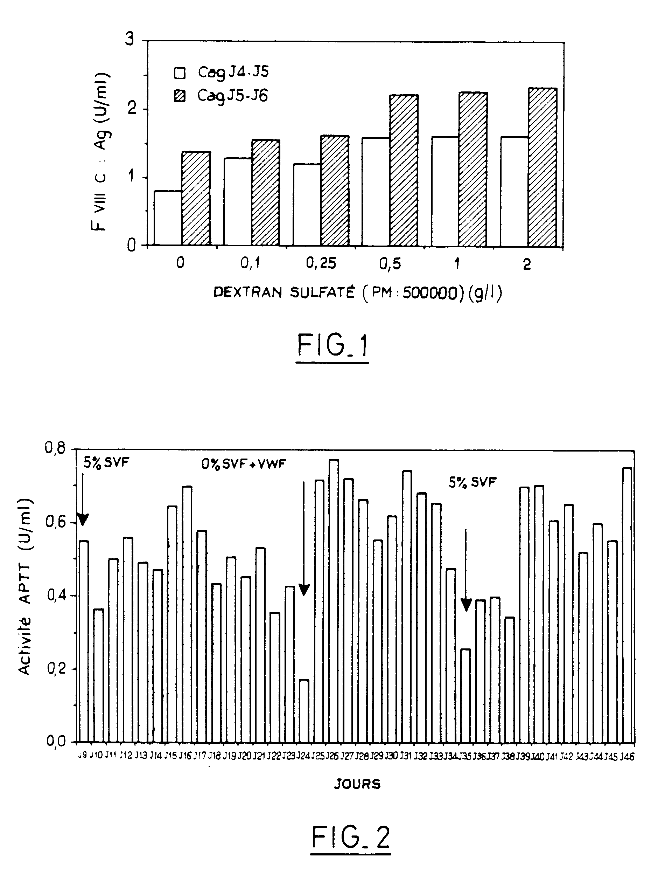

La figure 1 met en évidence l'existence d'un effet-dose du dextran sulfaté (Pharmacia PM : 500 000) sur l'expression du facteur VIII en plaques 6 puits.

On observe que sur 2 jours de production, J4-J5 et J5-J6, la production de facteur VIII est augmentée de manière significative par rapport à un témoin sans dextran sulfaté. Un seuil est atteint à 500mg/l de dextran sulfaté.

**EXEMPLE 6**

**Comparaison des effets du VWF et du dextran sulfaté (PM: 50 000) sur l'expression du facteur VIII dans un milieu chimiquement défini**

Cette étude est effectuée en biogénérateur 1 l (SGI). Le clone CHO TG 1566-3013 exprimant le facteur VIII complet est cultivé sur le support Cultispher-G à la concentration de 4 g/l (billes microporeuses de gélatine bovine réticulées).

Après une phase de croissance de 4 jours dans un milieu Iscove contenant 5% de SVF, on teste l'influence de 3 milieux de production différents :
- un milieu comportant 5% de SVF et ne comportant ni VWF ni dextran sulfaté (milieu A)
- un milieu sans SVF comportant 1 UI de VWF/ml (milieu B)
- un milieu sans VWF ni SVF et comportant 200 mg/l de dextran sulfaté (PM: 50 000) (milieu C).

Les résultats sont présentés à la figure 2.

L'activité en facteur VIII est exprimée en unités APTT/l de milieu dans un milieu qui ne contient pas de dextran sulfaté, et en unite Cag dans un milieu qui contient du dextran sulfaté (dosage ELISA). Le rapport entre les dosages APTT et ELISA est de l'ordre de 1.2 à 1.4.

On constate que les productions obtenues en milieu B sont supérieures à celles obtenues en milieu A (680± 67 U APTT/l contre 502± 96 U APTT/l). Les productions obtenues en milieu C sont comparables à celles obtenues en milieu B.

En conclusion, on montre clairement que le dextran sulfaté peut se substituer au facteur von Willebrand pour augmenter l'expression du facteur VIII dans un milieu chimiquement défini.

**Revendications**

1. Procédé de préparation de facteur VIII ou d'un analogue du facteur VIII par culture de cellules eucaryotes ayant incorporé un vecteur d'expression contenant un gène codant pour ledit facteur VIII ou un analogue de facteur VIII et séparation du facteur VIII ou de son analogue, caractérisé en ce que le milieu de culture contient au moins un dérivé d'un polymère polyanionique polysulfaté et en ce qu'il est exempt de sérum.

2. Procédé selon la revendication 1, caractérisé en ce que le polymère est un polymère polyosidique.

3. Procédé selon la revendication 2, caractérisé en ce que le polymère est un dérivé de polysaccharide.

4. Procédé selon la revendication 1, caractérisé en ce que le polymère est un polysaccharide sulfaté.

**5.** Procédé selon la revendication 1, caractérisé en ce que le dérivé polysulfaté est choisi de préférence parmi l'héparine, le dextran sulfaté et le sulfate d'hydroxyéthylamidon.

**6.** Procédé selon l'une des revendications 1 à 5, caractérisé en ce que le polymère présente un poids moléculaire compris entre 1000 et 1 000 000.

**7.** Procédé selon la revendication 6, caractérisé en ce qu'il s'agit d'un dextran sulfaté ayant un poids moléculaire compris entre 5000 et 700 000 et un degré de sulfatation de 0,5 à 18 % en poids de soufre.

**8.** Procédé selon la revendication 7, caractérisé en ce que le dextran sulfaté a de préférence un degré de sulfatation de 10 à 18 %.

**9.** Procédé selon les revendications 1 à 8, caractérisé en ce que l'analogue du facteur VIII préparé est le facteur VIII delta 2.

**10.** Procédé selon les revendications 1 à 8, caractérisé en ce que les cellules en culture sont des cellules de mammifères.

**11.** Procédé selon la revendication 9, caractérisé en ce que les cellules en culture sont des cellules CHO recombinées exprimant la molécule de facteur VIII ou analogue du facteur VIII de façon permanente.

**Claims**

**1.** Process for the preparation of factor VIII or an analogue of factor VIII by culturing eukaryotic cells having incorporated an expression vector containing a gene encoding the said factor VIII or an analogue of factor VIII and the separating the factor VIII or its analogue, characterized in that the culture medium contains at least one derivative of a polysulphated polyanionic polymer and in that it is free of serum.

**2.** Process according to Claim 1, characterized in that the polymer is a polyosidic polymer.

**3.** Process according to Claim 2, characterized in that the polymer is a polysaccharide derivative.

**4.** Process according to Claim 1, characterized in that the polymer is a sulphated polysaccharide.

**5.** Process according to Claim 1, characterized in that the polysulphated derivative is preferably chosen from among heparin, sulphated dextran and hydroxyethylstarch sulphate.

**6.** Process according to one of Claims 1 to 5, characterized in that the polymer has a molecular weight of between 1000 and 1,000,000.

**7.** Process according to Claim 6, characterized in that it is a sulphated dextran which has a molecular weight of between 5000 and 700,000 and a degree of sulphation of 0.5 to 18% by weight of sulphur.

**8.** Process according to Claim 7, characterized in that the sulphated dextran preferably has a degree of sulphation of 10 to 18%.

**9.** Process according to Claims 1 to 8, characterized in that the analogue of factor VIII which is prepared is factor VIII delta 2.

**10.** Process according to Claims 1 to 8, characterized in that the cells in culture are mammalian cells.

**11.** Process according to Claim 9, characterized in that the cells in culture are recombinant CHO cells which express the molecule of factor VIII or analogue of factor VIII in a continuous manner.

**Patentansprüche**

1. Verfahren zur Herstellung des Faktors VIII oder eines Analogons des Faktors VIII durch Kultivierung von Eukaryontenzellen, denen ein Expressionsvektor einverleibt worden ist, der ein Gen enthält, das für den genannten Faktor VIII oder ein Analogon des Faktors VIII codiert, und Abtrennung des Faktors VIII oder seines Analogons, dadurch gekennzeichnet, daß das Kulturmedium mindestens ein Derivat eines polysulfatierten polyanionischen Polymers enthält und frei von Serum ist.

2. Verfahren nach Anspruch 1, dadurch gekennzeichnet, daß das Polymer ein Polyosid-Polymer ist.

3. Verfahren nach Anspruch 2, dadurch gekennzeichnet, daß das Polymer ein Polysaccharidderivat ist.

4. Verfahren nach Anspruch 1, dadurch gekennzeichnet, daß das Polymer ein sulfatiertes Polysaccharid ist.

5. Verfahren nach Anspruch 1, dadurch gekennzeichnet, daß das polysulfatierte Derivat vorzugsweise ausgwählt wird aus der Gruppe Heparin, sulfatiertes Dextran und Hydroxyethylstärkesulfat.

6. Verfahren nach einem der Ansprüche 1 bis 5, dadurch gekennzeichnet, daß das Polymer ein Molekulargewicht zwischen 1000 und 1 000 000 hat.

7. Verfahren nach Anspruch 6, dadurch gekennzeichnet, daß es sich um ein sulfatiertes Dextran mit einem Molekulargewicht zwischen 5000 und 700 000 und einem Sulfatierungsgrad von 0,5 bis 18 Gew.-% Schwefel handelt.

8. Verfahren nach Anspruch 7, dadurch gekennzeichnet, daß das sulfatierte Dextran vorzugsweise einen Sulfatierungsgrad von 10 bis 18 % aufweist.

9. Verfahren nach den Ansprüchen 1 bis 8, dadurch gekennzeichnet, daß das hergestellte Analogon des Faktors VIII der Faktor VIII Delta 2 ist.

10. Verfahren nach den Ansprüchen 1 bis 8, dadurch gekennzeichnet, daß die Zellen in der Kultur Säugetierzellen sind.

11. Verfahren nach Anspruch 9, dadurch gekennzeichnet, daß die Zellen in der Kultur rekombinierte CHO-Zellen sind, die das Molekül des Faktors VIII oder des Analogons des Faktors VIII auf permanente Weise exprimieren.

FIG_1

FIG_2